# EUROPÄISCHE PATENTANMELDUNG

(11) **EP 2 275 408 A1**
(43) Veröffentlichungstag der Anmeldung: **19.01.2011**
(21) Anmeldenummer: 10179813.0
(22) Anmeldetag: 18.06.2008
(51) Int. Cl.: C07D 203/08

(54) **Verfahren zur Herstellung eines N-substituierten Aziridins**

(30) Priorität: 05.06.2008 EP 08157633; 20.06.2007 EP 07110690
(62) Teilanmeldung aus: 08761156.2
(71) Anmelder: BASF SE, 67056 Ludwigshafen (DE)
(72) Erfinder: Melder, Johann-Peter, 67459, Böhl-Iggelheim (DE); Ernst, Martin, 69121, Heidelberg (DE); Gerlach, Till, 67071, Ludwigshafen (DE); Schwab, Ekkehard, 67434, Neustadt (DE); Varszegi, Csaba, 3600, Genk (BE); Sels, Bert, 2490, Balen (BE); de Vos, Dirk, 3220, Holsbeek (BE)

(57) **Zusammenfassung**

Verfahren zur Herstellung eines N-substituierten Aziridins der Formel 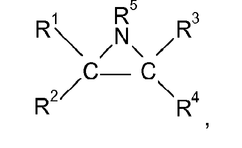 wobei man ein Olefin der Formel 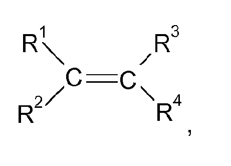 in denen R¹ bis R⁴ unabhängig voneinander Wasserstoff und R¹ bis R⁵ unabhängig voneinander lineare oder verzweigte Alkylreste mit 1 bis 16 Kohlenstoffatomen, Hydroxyalkylreste mit 1 bis 4 Kohlenstoffatomen, Cycloalkylreste mit 5 bis 7 Kohlenstoffatomen, Benzylreste und Phenylreste, die jeweils in o-, m- oder p-Stellung des Phenylrestes durch Methoxy-, Hydroxy-, Chlor- oder Alkylreste mit 1 bis 4 Kohlenstoffatomen substituiert sein können, darstellen und die Reste R¹ oder R² mit den Resten R³ oder R⁴ zu einem 5- bis 12-gliedrigen Ring geschlossen sein können oder die Reste R¹ und R² zu einem 5- bis 12-gliedrigen Ring geschlossen sein können, mit einem primären Amin der Formel R⁵NH₂ in Gegenwart von lod oder Brom umsetzt.

## Beschreibung

Die vorliegende Erfindung betrifft Verfahren zur Herstellung eines N-unsubstituierten oder N-substituierten Aziridins.

N-unsubstituierte Aziridine und N-substituierte Aziridine stellen wichtige organische Zwischenprodukte dar, die eine hohe Reaktivität besitzen und z. B. zur Herstellung von Polymeren und Heterocyclen dienen.

Aziridin (C₂H₅N) wird großtechnisch durch Epoxidation von Ethylen mit Luft oder Sauerstoff zu Ethylenoxid, dessen Ringöffnung mit Ammoniak zu einem Gemisch aus Mono-, Di- und Triethanolamin, Abtrennung von Ethanolamin aus diesem Gemisch, Veresterung von Ethanolamin mit Schwefelsäure zu beta-Aminoethylschwefelsäure und dessen Cyclisierung zu Aziridin hergestellt. Dabei werden pro Mol Aziridin zwei Mole Natronlauge verwendet, aus denen ein Mol Natriumsulfat entsteht (H. J. Arpe, Industrielle Organische Chemie, 6. Auflage 2007, Wiley-VCH-Verlag, Seiten 158 bis 160 und 172 bis 174). In ähnlicher Weise sind auch substituierte Aziridine zugänglich.

Nachteilig an diesen Verfahren sind die zahlreichen Verfahrensschritte und der stöchiometrische Salzanfall.

Bekannt ist, am Stickstoff durch p-Toluolsulfonylreste substituierte Aziridine durch Umsetzung von Olefinen mit Chloramin T (zugänglich aus p-Toluolsulfonamid und Natriumhypochlorit), Kaliumcarbonat, Siliziumdioxid und katalytischen Mengen Iod herzustellen (S. Minakata et al., Angew. Chem. int. Ed. 2004, 43, Seiten 79 bis 81).

Nachteilig dabei ist, dass der Aziridinstickstoff durch das in mehrstufiger Synthese herstellbare Chloramin T eingebracht wird und daher stöchiometrische Mengen Chloramin T benötigt werden. Es bedeutet auch, dass nur N-substituierte Aziridine zugänglich sind und stöchiometrische Mengen Natriumchlorid anfallen.

Es ist weiterhin bekannt, am Stickstoff durch p-Toluolsulfonylreste substituierte Aziridine durch Umsetzung von Olefinen mit p-Toluolsulfonamid und in situ aus tert.-Butylhypochlorit und Natriumiodid hergestelltem tert.-Butylhypoiodit zu synthetisieren (S. Minakata et al., Chem. Commun. 2006, Seiten 3337 bis 3339 und JP-A-2007 055958).

Diese Methode besitzt ähnliche Nachteile wie das mit Chloramin T beschriebene Verfahren.

Es bestand daher die Aufgabe, Nachteile aus dem Stand der Technik zu überwinden und ein Verfahren aufzufinden, das es ermöglicht, N-unsubstituierte und N-substituierte Aziridine aus Olefinen in wenigen Reaktionsschritten, möglichst ohne oder mit nur geringem Salzanfall, herzustellen.

Erfindungsgemäß wurde erkannt, dass es wesentlich vorteilhafter wäre, den Aziridinstickstoff über Ammoniak oder einem primären Amin einzubringen.

Demgemäß wurde ein Verfahren zur Herstellung eines N-unsubstituierten Aziridins der Formel II 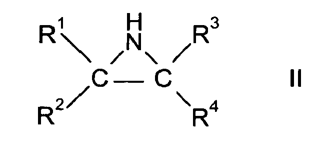 gefunden, welches dadurch gekennzeichnet ist, dass man ein Olefin der Formel I 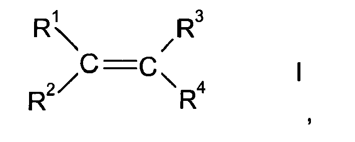 in der R¹ bis R⁴ unabhängig voneinander Wasserstoff, lineare oder verzweigte Alkylreste mit 1 bis 16 Kohlenstoffatomen, Hydroxyalkylreste mit 1 bis 4 Kohlenstoffatomen, Cycloalkylreste mit 5 bis 7 Kohlenstoffatomen, Benzylreste und Phenylreste, die jeweils in o-, m- oder p-Stellung des Phenylrestes durch Methoxy-, Hydroxy-, Chlor- oder Alkylreste mit 1 bis 4 Kohlenstoffatomen substituiert sein können, darstellen und die Reste R¹ oder R² mit den Resten R³ oder R⁴ zu einem 5- bis 12-gliedrigen Ring geschlossen sein können oder die Reste R¹ und R² zu einem 5- bis 12-gliedrigen Ring geschlossen sein können, mit Ammoniak in Gegenwart von Iod oder Brom umsetzt.

Weiterhin wurde ein Verfahren zur Herstellung eines N-unsubstituierten Aziridins der Formel II gefunden, welches dadurch gekennzeichnet ist, dass man ein Olefin der Formel I mit Ammoniak in Gegenwart eines Iodids und eines Oxidationsmittels, das das Iodid zu Iod zu oxidieren vermag, umsetzt.

Weiterhin wurde ein Verfahren zur Herstellung eines N-unsubstituierten Aziridins der Formel II gefunden, welches dadurch gekennzeichnet ist, dass man ein Olefin der Formel I mit Ammoniak in Gegenwart eines Bromids und eines Oxidationsmittels, das das Bromid zu Brom zu oxidieren vermag, umsetzt.

Weiterhin wurde ein Verfahren zur Herstellung eines N-substituierten Aziridins der Formel III 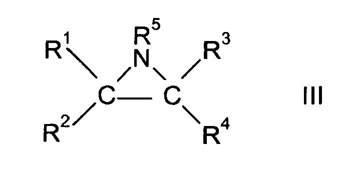 gefunden, welches dadurch gekennzeichnet ist, dass man ein Olefin der Formel I 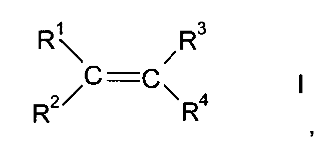 wobei R¹ bis R⁴ unabhängig voneinander Wasserstoff und R¹ bis R⁵ unabhängig voneinander lineare oder verzweigte Alkylreste mit 1 bis 16 Kohlenstoffatomen, Hydroxyalkylreste mit 1 bis 4 Kohlenstoffatomen, Cycloalkylreste mit 5 bis 7 Kohlenstoffatomen, Benzylreste und Phenylreste, die jeweils in o-, m- oder p-Stellung des Phenylrestes durch Methoxy-, Hydroxy-, Chlor- oder Alkylreste mit 1 bis 4 Kohlenstoffatomen substituiert sein können, darstellen und die Reste R¹ oder R² mit den Resten R³ oder R⁴ zu einem 5- bis 12-gliedrigen Ring geschlossen sein können oder die Reste R¹ und R² zu einem 5- bis 12-gliedrigen Ring geschlossen sein können, mit einem primären Amin der Formel R⁵NH₂ in Gegenwart von Iod oder Brom umsetzt, wobei die Konzentration des primären Amins (R⁵NH₂) im Reaktionsgemisch kleiner oder gleich 1,1 molar (≤ 1,1 M) beträgt.

Weiterhin wurde ein Verfahren zur Herstellung eines Aziridins der Formel III gefunden, welches dadurch gekennzeichnet ist, dass man ein Olefin der Formel I mit einem primären Amin der Formel R⁵NH₂ in Gegenwart eines Iodids und eines Oxidationsmittels, das das Iodid zu Iod zu oxidieren vermag, umsetzt, wobei die Konzentration des primären Amins (R⁵NH₂) im Reaktionsgemisch kleiner oder gleich 1,1 molar (≤ 1,1 M) beträgt.

Weiterhin wurde ein Verfahren zur Herstellung eines Aziridins der Formel III gefunden, welches dadurch gekennzeichnet ist, dass man ein Olefin der Formel I mit einem primären Amin der Formel R⁵NH₂ in Gegenwart eines Bromids und eines Oxidationsmittels, das das Bromid zu Brom zu oxidieren vermag, umsetzt, wobei die Konzentration des primären Amins (R⁵NH₂) im Reaktionsgemisch kleiner oder gleich 1,1 molar (≤ 1,1 M) beträgt.

Im Verfahren zur Herstellung eines N-unsubstituierten Aziridins der Formel II beträgt die Konzentration des Ammoniaks im Reaktionsgemisch bei Reaktionsbeginn bevorzugt größer oder gleich 1,2 molar (≥ 1,2 M), insbesondere größer oder gleich 1,25 molar (≥ 1,25 M), z. B. im Bereich von ≥ 1,2 bis 15 molar, besonders im Bereich von ≥ 1,2 bis 2 molar.

Im Verfahren zur Herstellung eines N-substituierten Aziridins der Formel III beträgt die Konzentration des primären Amins (R⁵NH₂) im Reaktionsgemisch bevorzugt kleiner oder gleich 1,0 molar (≤ 1,0 M). Die Konzentration des primären Amins (R⁵NH₂) im Reaktionsgemisch beträgt bei Reaktionsbeginn bevorzugt größer als 0,5 molar (> 0,5 M), besonders größer als 0,7 molar (> 0,7 M), ganz besonders größer als 0,8 molar (> 0,8 M).

Erfindungsgemäß wurde erkannt, dass das Verfahren zur Herstellung eines N-substituierten Aziridins der Formel III nur dann besonders vorteilhaft, insbesondere hinsichtlich Ausbeute und Selektivität, verläuft, wenn eine anfängliche Konzentration des primären Amins (R⁵NH₂) im Reaktionsgemisch in den o.g. Bereichen (> 0,5 bis ≤ 1,1 M, besonders > 0,8 bis ≤ 1,1 M), eingestellt wird.

Die erfindungsgemäße Umsetzung lässt sich z. B. bei Verwendung von Styrol als Olefin und Ammoniak (und Wasser als Lösungsmittel), durch die folgende Reaktionsgleichung darstellen:

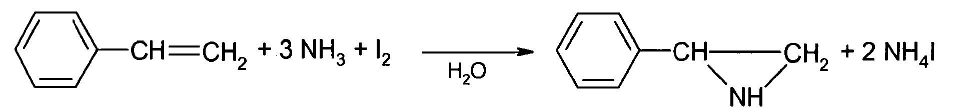

Die bevorzugte Ausführungsform der Verfahren mit Iodiden und Oxidationsmitteln lässt sich z. B. bei Verwendung von Styrol als Olefin, Ammoniak, Wasser als Lösungsmittel, Ammoniumiodid als Iodid und Natriumhypochlorit als Oxidationsmittel durch die folgende Reaktionsgleichung darstellen:

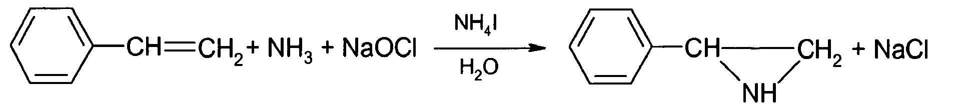

Entsprechendes gilt bei Verwendung eines primären Amins (R⁵NH₂) anstelle von Ammoniak.

Beispiele für die Reste R¹ bis R⁴ in den Olefinen der Formel I sind wie folgt:
H, Methyl, Ethyl, n-Propyl, iso-Propyl, n-Butyl, iso-Butyl, n-Pentyl, n-Hexyl, n-Heptyl, n-Octyl, n-Nonyl, n-Decyl, 3-Hydroxypropyl, 4-Hydroxybutyl, Cyclopentyl, Cyclohexyl.

Beispiele für geeignete Olefine I sind Ethylen, Propylen, i-Buten, 1-Buten, 2-Buten, 1-Penten, 1-Hexen, 2-Hexen, Cyclopenten, Methylencyclopentan, Cyclohexen, Methylencyclohexan, 3-Hexen, 2-Methyl-1-hepten, 1-Octen, Cycloocten, 2-Octen, 1-Dodecen, Styrol, alpha-Methylstyrol, beta-Methylstyrol, p-Methylstyrol, p-Methoxystyrol, p-Hydroxystyrol, m-Chlorstyrol, p-Chlorstyrol, 2-Buten-1-ol, 2-Buten-1,4-diol.

Ammoniak wird bevorzugt als wässrige Lösung eingesetzt, die bevorzugt 0,1 bis 30 Gew.-% Ammoniak enthalten kann. Die erfindungsgemäße Umsetzung kann auch in Gegenwart von Verbindungen durchgeführt werden, die unter den Reaktionsbedingungen Ammoniak freizusetzen vermögen.

Beispiele für die Reste R⁵ in dem primären Amin (R⁵NH₂) sind wie folgt:
Methyl, Ethyl, n-Propyl, iso-Propyl, n-Butyl, iso-Butyl, n-Pentyl, n-Hexyl, n-Heptyl, n-Octyl, n-Nonyl, n-Decyl, Cyclopentyl, Cyclohexyl.

Besonders bevorzugte primäre Amine (R⁵NH₂) sind Methylamin und Ethylamin.

Das primäre Amin (R⁵NH₂) wird bevorzugt als wässrige Lösung eingesetzt. Die Lösung kann bis zur Sättigungslöslichkeit primäres Amin enthalten. Die erfindungsgemäße Umsetzung kann auch in Gegenwart von Verbindungen durchgeführt werden, die unter den Reaktionsbedingungen das primäre Amin freizusetzen vermögen.

In einer Ausführungsform der Verfahren können Iod und/oder Iodide verwendet werden. Anstelle von Iod und Iodiden können auch Brom und Bromide eingesetzt werden. Iod und Iodide sind dabei gegenüber Brom und Bromiden bevorzugt.

Als Iodide oder Bromide sind Alkalimetall-, Erdalkalimetall-, Ammonium- und Tetraalkylammonium-iodide bzw. Alkalimetall-, Erdalkalimetall-, Ammonium- und Tetraalkylammonium-bromide, wobei die Alkylreste in den Alkylammoniumhalogeniden bevorzugt jeweils und unabhängig voneinander 1 bis 5 Kohlenstoffatome enthalten, und N-Halogenimide geeignet.

Beispiele für solche Halogenide sind: Ammoniumiodid, Ammoniumbromid, N-Bromsuccinimid, N-Iod-succinimid, Natriumiodid, Natriumbromid, Kaliumiodid, Kaliumbromid, Magnesiumiodid, Magnesiumbromid, Tetramethylammoniumiodid, Tetramethylammoniumbromid; besonders bevorzugt sind Ammoniumiodid und Ammoniumbromid.

Es ist auch möglich, anstelle von Iodiden Gemische aus Iodiden und elementarem Iod und anstelle von Bromiden Gemische aus elementarem Brom und Bromiden einzusetzen.

Bei Gemischen aus Iodid und Iod kann das Molverhältnis von Iodid zu Iod 1 : 0,01 bis 0,01 : 1 betragen. Das gleiche Molverhältnis gilt für Bromide und Brom.

In den erfindungsgemäßen Verfahren eingesetzte Oxidationsmittel sind in der Lage, Iodide zu Iod bzw. Bromide zu Brom zu oxidieren. Geeignet sind beispielsweise Sauerstoff, z. B. in Form von Luft, Wasserstoffperoxid, bevorzugt als wässrige Lösung, Alkylhydroperoxide wie beispielsweise Cumolhydroperoxid, tert.-Butylhydroperoxid, Cyclohexylhydroperoxid, Methylphenylhydroperoxid, Anthrachinonendoperoxid, unterchlorige Säure, Alkali- und Erdalkalihypochlorite, tert.-Butylhypochlorit, tert.-Butylhypobromit, tert.-Butylhypoiodit und Distickstoffmonoxid.

Es ist weiterhin möglich, Iodide oder Bromide elektrochemisch zu Iod bzw. Brom zu oxidieren.

Als Lösungsmittel wird bevorzugt Wasser verwendet, in dem sich Ammoniak bzw. primäres Amin, Iodide bzw. Bromide und die Mehrzahl der Oxidationsmittel ausreichend Iösen. Iod ist zwar in Wasser nur in sehr geringen Mengen, in Gegenwart von Iodiden jedoch leicht löslich.

Es kann aber auch vorteilhaft sein, Gemische aus Wasser und organischen Lösungsmitteln zu verwenden, die unter den Reaktionsbedingungen inert sind. Dabei kommen gut wasserlösliche und weniger gut wasserlösliche Lösungsmittel in Frage. Zu den gut wasserlöslichen Lösungsmitteln gehören z. B. Ether wie Tetrahydrofuran und Dioxan, zu den weniger gut löslichen aliphatische, cycloaliphatische und aromatische Kohlenwasserstoffe wie z. B. n-Hexan, Heptan, Cyclohexan und Toluol.

Der Zusatz von wenig wasserlöslichen Lösungsmitteln führt im Allgemeinen zu einer verbesserten Trennung von organischer und wässriger Phase und damit einer vereinfachten Aufarbeitung des Reaktionsgemisches.

Das Molverhältnis von Olefin (I) zu Ammoniak zu Iodid, Iod oder Iodid + Iod beträgt bevorzugt 1 : 1 bis 100 : 0,001 bis 1,5, besonders bevorzugt 1 : 1 bis 90 : 0,01 bis 1,3, ganz besonders bevorzugt 1 : 1 bis 80 : 0,1 bis 1,1. Die gleichen Molverhältnisse gelten für das Verhältnis von Olefin zu Ammoniak zu Bromid, Brom oder Bromid + Brom.

Das Molverhältnis von Olefin (I) zu primärem Amin (R⁵NH₂) zu Iodid, Iod oder Iodid + Iod beträgt bevorzugt 1 : 1 bis 100 : 0,001 bis 1,5, besonders bevorzugt 1 : 1 bis 90 : 0,01 bis 1,3, ganz besonders bevorzugt 1 : 1 bis 80 : 0,1 bis 1,1.

Die gleichen Molverhältnisse gelten für das Verhältnis von Olefin zu primärem Amin (R⁵NH₂) zu Bromid, Brom oder Bromid + Brom.

Das Molverhältnis von Iodid bzw. Iod zu Oxidationsmittel beträgt bevorzugt 1 : 1 bis 10, besonders bevorzugt 1 : 1 bis 4, ganz besonders bevorzugt 1 : 1 bis 3.

Das Molverhältnis von Olefin (I) zu Oxidationsmittel beträgt bevorzugt 1 : 1 bis 5, besonders bevorzugt 1 : 1 bis 3, ganz besonders bevorzugt 1 : 2.

Der Reaktionsansatz enthält bevorzugt 30 bis 90 Gew.-% Wasser und 1 bis 30 Gew.-% organisches Lösungsmittel, besonders bevorzugt 70 bis 80 Gew.-% Wasser und 2 bis 20 Gew.-% organisches Lösungsmittel.

In einer bevorzugten Fahrweise wird dem Reaktionsansatz eine oberflächenaktive Substanz hinzugefügt, die eine deutliche Steigerung der Aziridin-Ausbeute bewirkt.

Als oberflächenaktive Substanzen sind grundsätzlich alle Stoffgruppen geeignet, die in Ullmanns Encyclopedia of Industrial Chemistry, 6. Auflage, Band 35, Stichwort "surfactants", Seiten 293 bis 368 genannt sind.

Dazu gehören anionische, kationische, nichtionische, amphotere und Anion/Kationoberflächenaktive Substanzen ("Tenside").

Bevorzugte oberflächenaktive Substanzen sind dabei nichtionische Tenside, wie z. B. Polyalkylenglykolalkylether (z. B. Brij ®). Sie stellen Copolymere dar, wobei der lipophile Teil aus Fettalkoholen, der hydrophile Teil aus kurzkettigen Polyalkylenglykolen, bevorzugt Polyethylenglykolen, besteht.

Als Fettalkohole werden bevorzugt die sich von Laurin-, Palmitin-, Stearin- oder Ölsäure ableitenden Alkohole verwendet.

Weitere Beispiele für geeignete nichtionische Tenside sind:
Tritone ® (Ethoxylate des 4-(1.1.3.3-Tetramethylbutyl)phenols),
Lutensole ® (ethoxylierte Fettalkohole, Alkylphenole oder Fettamine),
Tweene ® (Polyoxyethylenderivate der Sorbitanester wie Polyethoxysorbitanlaurat).

Die Menge an oberflächenaktiven Substanzen beträgt bevorzugt, jeweils bezogen auf das gesamte Reaktionsgemisch, 0,01 bis 10, besonders bevorzugt 0,5 bis 5, ganz besonders bevorzugt 1 bis 2 Gew.-%.

Anstelle von oberflächenaktiven Substanzen oder zusätzlich zu ihnen kann in Gegenwart von Zeolithen und/oder anderen porösen anorganischen Materialien gearbeitet werden. Auch hier kann man eine deutliche Steigerung der Aziridin-Ausbeuten beobachten.

Als Zeolithe sind grundsätzlich alle natürlich vorkommenden und synthetisch herstellbaren Zeolithe geeignet, d. h. Zeolithe der Typen A, X, Y und L, die sich in den Porenweiten und dem Verhältnis von SiO₂ zu Al₂O₃ (Modul) unterscheiden.

Bevorzugt sind aus SiO₂ bestehende Zeolithe wie z. B. Silikalit und Zeolithe mit einem hohen SiO₂-Gehalt d. h. hohem Modul, wie z. B. ZSM-5-Zeolith (Modul ca. 30) und synthetischer Mordenit (Modul ca. 10).

Die Menge an Zeolith und/oder anderen porösen anorganischen Materialien beträgt bevorzugt, jeweils bezogen auf das gesamte Reaktionsgemisch, 1 bis 20, besonders bevorzugt 1 bis 10, ganz besonders bevorzugt 1 bis 5 Gew.-%.

Die Herstellung der Aziridine erfolgt bevorzugt bei Temperaturen im Bereich von 0 °C bis 300 °C, besonders bevorzugt bei 10 °C bis 250 °C, ganz besonders bevorzugt bei 20 °C bis 200 °C, z. B. bevorzugt im Bereich von 20 bis 50 °C.

Gearbeitet wird bevorzugt bei einem Absolutdruck im Bereich von 1 bar bis 300 bar, besonders bevorzugt 1 bar bis 250 bar, ganz besonders bevorzugt 1 bis 150 bar, z. B. bevorzugt im Bereich von 1 bis 10 bar.

Die erfindungsgemäße Umsetzung kann einstufig, zweistufig oder mehr als zweistufig, in der Flüssigphase, durchgeführt werden.

Bei einstufiger Arbeitsweise werden die Reaktionspartner Olefin, Ammoniak bzw. primäres Amin, Halogen und/oder Halogenide in Gegenwart eines Oxidationsmittels in Wasser als Lösungsmittel und gegebenenfalls zusätzlich in Anwesenheit eines organischen Lösungsmittels, einer oberflächenaktiven Substanz und/oder eines suspendiertem oder fest angeordneten Zeolithen in einem Reaktionsgefäß unter den angegebenen Reaktionsbedingungen z. B. 0,1 bis 30 Stunden lang durchmischt. Die Umsetzung kann diskontinuierlich oder kontinuierlich durchgeführt werden. Im Allgemeinen erfolgt nach der Umsetzung eine Trennung des Reaktionsaustrags in eine flüssige wässrige und eine flüssige organische Phase.

Die flüssige organische Phase enthält die gebildeten Aziridine und gegebenenfalls nicht umgesetzte Olefine, oberflächenaktive Substanzen und organische Lösungsmittel.

Die flüssige wässrige Phase enthält Halogen und Halogenid, Ammoniak bzw. primäres Amin und gegebenenfalls oberflächenaktive Substanzen. Sie können in die Synthesestufe zurückgeführt werden.

Die Aufarbeitung der organischen Phase kann in an sich bekannter Weise, z. B. durch Destillation, erfolgen. Nicht umgesetztes Olefin, organische Lösungsmittel und oberflächenaktive Substanzen können in die Synthesestufe zurückgeführt werden.

In einer vorteilhaften Variante des erfindungsgemäßen Verfahrens werden die bei der Aziridin-Herstellung entstehenden Iodide bzw. Bromide nachfolgend oxidiert und in die Synthesestufe zurückgeführt:
Bei zweistufiger Arbeitsweise werden im ersten Schritt die Reaktionspartner Olefin, Ammoniak bzw. primäres Amin und Halogen (d.h. Brom oder Iod) ohne Zugabe eines Oxidationsmittels in Wasser als Lösungsmittel und gegebenenfalls zusätzlich in Anwesenheit eines organischen Lösungsmittels, einer oberflächenaktiven Substanz und/oder eines suspendierten oder fest angeordneten Katalysators, d.h. den oben beschriebenen Zeolithen und/oder anderen porösen anorganischen Materialien, in einem Reaktionsgefäß unter den angegebenen Reaktionsbedingungen, z. B. 0,1 bis 30 Stunden lang, durchmischt. Die Umsetzung kann diskontinuierlich oder kontinuierlich durchgeführt werden. Nach der Umsetzung werden die Phasen getrennt. Die organische Phase wird, wie bei der einstufigen Fahrweise beschrieben, aufgearbeitet.

Die wässrige Phase wird im zweiten Schritt mit einem Oxidationsmittel, z.B. einem Oxidationsmittel wie oben beschrieben, behandelt oder elektrochemisch oxidiert. Dabei werden Iodid bzw. Bromid zu Iod bzw. Brom oxidiert. Die halogenhaltige wässrige Phase wird dann in die Synthesestufe zurückgeführt.

### Beispiele

Die Zusammensetzung der Reaktionsausträge und die Ausbeuten und Selektivitäten der Aziridine wurden gaschromatographisch ermittelt. Brij 35 ® ist der Handelsname für Polyoxyethylen(23)laurylether.

Triton ® X-100 stellt ein nichtionogenes Tensid dar, das aus Ethoxylaten des 4-(1.1.3.3-Tetramethylbutyl)phenols besteht. - Lutensole ® sind nichtionische Tenside auf der Basis ethoxylierter Fettalkohole, Alkylphenole oder Fettamine. - Tweene® sind Polyoxyethylenderivate der Sorbitanester wie Polyethoxysorbitanlaurat (Tween ® 20), Polyethoxysorbitanpalmitat (Tween ® 40) und Polyethoxysorbitanoleat (Tween ® 80).

### Beispiel 1

Brij 35 ® (90 mg) und 0,5 mmol Iod (127 mg) wurden zu 5 ml einer 25 gew.-%igen wässrigen Ammoniak-Lösung hinzugefügt. Die Umsetzung wurde durch Zugabe von 0,5 mmol Styrol (57 µl) gestartet. (Der Anteil an Brj 35 betrug also 2 Gew.-%, die Molarität an Iod und Styrol jeweils 0,1 M). Nach 2 Stunden Reaktionszeit bei Raumtemperatur wurde das Reaktionsgemisch mit Diethylether extrahiert. Die Ausbeute an 2-Phenylaziridin betrug 65 % (Selektivität > 99 %).

Wurde die Umsetzung unter gleichen Bedingungen 24 Stunden lang durchgeführt, betrug die Ausbeute 81 % (Selektivität 99 %).

### Beispiel 2

Brij 35 ® (180 mg) und 0,5 mmol Iod (127 mg) wurden zu 5 ml einer 25 gew.-%igen, wässrigen Ammoniak-Lösung hinzugefügt. Die Umsetzung wurde durch Zugabe von 0,49 mmol alpha-Methylstyrol (65 µl) gestartet. (Der Anteil an Brj 35 betrug also 4 Gew.-%, die Molarität an Iod und alpha-Methylstyrol jeweils 0,1 M). Nach 2 Stunden Reaktionszeit bei Raumtemperatur wurde das Reaktionsgemisch mit Diethylether extrahiert. Die Ausbeute an 2-Methyl-2-phenylaziridin betrug 76 % (Selektivität > 99 %).

### Beispiel 3

a) Brij 35 ® (180 mg) und 0,5 mmol Iod (127 mg) wurden zu 5 ml einer 25 gew.- %igen wässrigen Ammoniak-Lösung hinzugefügt. Die Umsetzung wurde durch Zugabe von 0,5 mmol p-Chlorstyrol (60 µl) gestartet. (Der Anteil an Brj 35 betrug also 4 Gew.-%, die Molarität an Iod und p-Chlorstyrol jeweils 0,1 M). Nach 2 Stunden Reaktionszeit bei Raumtemperatur wurde das Reaktionsgemisch mit Diethylether extrahiert. Die Ausbeute an 2-(p-Chlorphenyl)aziridin betrug 60 % (Selektivität > 99 %).
b) Unter gleichen Reaktionsbedingungen wie in Beispiel 3 a) wurden beta-Methylstyrol, p-Methylstyrol, p-Methoxystyrol und m-Chlorstyrol zu den entsprechenden Aziridinen umgesetzt. Folgende Ausbeuten wurden erhalten: 1-Phenyl-2-methylaziridin 30 %, 2-(p-Methylphenyl)aziridin 60 %, 2-(p-Methoxyphenyl)aziridin 56 %, 2-(m-Chlorphenyl)aziridin 61 %.
c) Unter gleichen Reaktionsbedingungen wie in Beispiel 3 a) wurde Styrol in Gegenwart von einer Reihe von ungeladenen oberflächenaktiven Substanzen (4 Gew.-%) umgesetzt. Die Ausbeuten an 2-Phenylaziridin sind hinter den oberflächenaktiven Substanzen in Klammern angegeben: Triton X-100 (45 %), Lutensole, AT25 (49 %), FB AT80 (50 %), XL 140 (51 %), Tween 20 (49 %), 40 (51 %), 80 (49 %).
d) Wurde Beispiel 3 c) in Abwesenheit einer oberflächenaktiven Substanz durchgeführt, wurde 1 % 2-Phenylaziridin gefunden.

### Beispiel 4

a) Wurde in Beispiel 3 c) anstelle von oberflächenaktiven Substanzen in Gegenwart von Silikalit (5,5 Gew.-%) gearbeitet, so betrug die Ausbeute an 2-Phenylaziridin 35 %.
b) Wurde in Beispiel 4 a) anstelle von Silikalit MCM-41 (5,5 Gew.-%) eingesetzt, so betrug die Ausbeute 35 %.

### Beispiel 5

Brij 35 ® (180 mg) und 0,5 mmol Iod (127 mg) wurden zu 5 ml einer 25 gew.-%igen wässrigen Ammoniak-Lösung hinzugefügt. Die Umsetzung wurde durch Zugabe von 0,46 mmol 2-Methyl-1-hepten (78 µl) gestartet. (Der Anteil an Brj 35 betrug also 4 Gew.-%, die Molarität an Iod 0,1 M und an 2-Methyl-1-hepten 0,9 M). Nach 2 Stunden Reaktionszeit bei 70 °C wurde das Reaktionsgemisch mit Diethylether extrahiert. Die Ausbeute an 2-Methyl-2-pentylaziridin betrug 1 % (Selektivität > 98 %).

### Beispiel 6

Brij 35 ® (180 mg), Ammoniumiodid (73 mg) und 0,5 mmol Styrol (57 µl) wurden zu 5 ml einer 25 gew.-%igen wässrigen Ammoniak-Lösung hinzugefügt. Die Umsetzung wurde durch die portionsweise Zugabe einer 10 bis 13 gew.-%igen wässrigen Natriumhypochlorit-Lösung (400 µl) gestartet, wobei jeweils 40 µl pro 5 Minuten zugegeben wurden. Nach 2 Stunden Reaktionszeit bei Raumtemperatur wurde das Reaktionsgemisch mit Diethylether extrahiert. Die Ausbeute an 2-Phenylaziridin betrug 74 % (Selektivität 98 %).

### Beispiel 7

Brij 35 ® (180 mg), Ammoniumiodid (73 mg) und 0,46 mmol 2-Methyl-1-hepten (78 µl) wurden zu 5 ml einer 25 gew.-%igen wässrigen Ammoniak-Lösung hinzugefügt. Die Umsetzung wurde durch die portionsweise Zugabe einer 10 bis 13 gew.-%igen wässrigen Natriumhypochlorit-Lösung (1 ml) gestartet. Die Natriumhypochlorit-Lösung wurde in Teilmengen von 200 µl pro 5 Minuten zugegeben. Nach 2 Stunden Reaktionszeit bei Raumtemperatur wurde das Reaktionsgemisch mit Diethylether extrahiert. Die Ausbeute an 2-Methyl-2-pentyl-aziridin betrug 1 %.

### Beispiel 8 (Vergleichsbeispiel)

Brij 35 ® (180 mg) und 0,5 mmol Styrol (57 µl) wurden zu 5 ml einer 25 gew.-%igen wässrigen Ammoniak-Lösung hinzugefügt. Die Umsetzung wurde durch die portionsweise Zugabe einer 10 bis 13 gew.-%igen wässrigen Natriumhypochlorit-Lösung (1 ml) gestartet, wobei jeweils 200 µl pro 5 Minuten zugegeben wurden. Nach 2 Stunden Reaktionszeit bei Raumtemperatur wurde das Reaktionsgemisch mit Diethylether extrahiert. Die Ausbeute an 2-Phenylaziridin betrug < 1 %.

### Beispiel 9

Brij 35 ® (180 mg), Ammoniumiodid (50 mol-%, 37 mg) und 0,5 mmol Styrol (57 µl) wurden zu 5 ml einer 25 %igen wässrigen Ammoniaklösung hinzugefügt. Die Reaktion wurde durch die portionsweise Zugabe (40 µl/5 Min.) einer 10 bis 13 gew.-%igen wässrigen Lösung von Natriumhypochlorit (400 µl) gestartet. Nach zwei Stunden Reaktionszeit bei Raumtemperatur wurde das Reaktionsgemisch mit Diethylether extrahiert. Die Ausbeute an 2-Phenylaziridin betrug 60 %.

### Beispiel 10

Brij 35 ® (180 mg), Ammoniumiodid (29 mg) und 0,5 mmol Styrol (57 µl) wurden zu 5 ml einer 1,3 molaren wässrigen Ammoniak-Lösung hinzugefügt. Die Umsetzung wurde durch die portionsweise Zugabe einer 10 bis 13 gew.-%igen wässrigen Natriumhypochlorit-Lösung (400 µl) gestartet, wobei jeweils 40 µl pro 5 Minuten zugegeben wurden. Nach 2 Stunden Reaktionszeit bei Raumtemperatur wurde das Reaktionsgemisch mit Diethylether extrahiert. Die Ausbeute an 2-Phenylaziridin betrug 90 % (Selektivität 99 %).

### Beispiel 11

Brij 35 ® (180 mg), Ammoniumiodid (29 mg) und 0,5 mmol (Menge siehe unten) eines substituierten Styrolderivates wurden zu 5 ml einer 1,3 molaren wässrigen Ammoniak-Lösung hinzugefügt. Die Umsetzung wurde durch die portionsweise Zugabe einer 10 bis 13 gew.-%igen wässrigen Natriumhypochlorit-Lösung (400 µl) gestartet, wobei jeweils 40 µl pro 5 Minuten zugegeben wurden. Nach 2 Stunden Reaktionszeit bei Raumtemperatur wurde das Reaktionsgemisch mit Diethylether extrahiert. Die Ausbeute an den einzelnen substituierten 2-Phenylaziridinen betrug:
74 % 2-(p-Chlorphenyl)-aziridin (Selektivität 99 %) aus p-Chlorstyren (60 µl)
92 % 2-Methyl-2-phenyl-aziridin (Selektivität 99 %) aus alpha-Methylstyren (65 µl)
56 % 2-Methyl-3-phenyl-aziridin (Selektivität 98 %) aus beta-Methylstyren (65 µl)

### Beispiel 12

Brij 35 ® (90 mg) und 0,5 mmol Iod (127 mg) wurden zu 5 ml einer 1 molaren wässrigen Methylamin-Lösung hinzugefügt. Die Umsetzung wurde durch Zugabe von 0,5 mmol Styrol (57 µl) gestartet. Nach 2 Stunden Reaktionszeit bei Raumtemperatur wurde das Reaktionsgemisch mit Diethylether extrahiert. Die Ausbeute an 1-Methyl-2-phenylaziridin betrug 43 % (Selektivität 84 %).

Wurde die Umsetzung unter gleichen Bedingungen 5 h Stunden lang durchgeführt, betrug die Ausbeute 64 % (Selektivität 86 %).

### Beispiel 13

Brij 35 ® (90 mg) und 0,5 mmol Iod (127 mg) wurden zu 5 ml einer 0,6 molaren wässrigen Ethylamin-Lösung hinzugefügt. Die Umsetzung wurde durch Zugabe von 0,5 mmol Styrol (57 µl) gestartet. Nach 2 Stunden Reaktionszeit bei Raumtemperatur wurde das Reaktionsgemisch mit Diethylether extrahiert. Die Ausbeute an 1-Ethyl-2-phenylaziridin betrug 28 % (Selektivität 93 %).

### Beispiel 14

Brij 35 ® (90 mg) und 0,5 mmol Iod (127 mg) wurden zu 5 ml einer 1 molaren wässrigen Methylamin-Lösung hinzugefügt. Die Umsetzung wurde durch Zugabe von 0,5 mmol p-Chlorstyrol (60 µl) gestartet. Nach 2 Stunden Reaktionszeit bei Raumtemperatur wurde das Reaktionsgemisch mit Diethylether extrahiert. Die Ausbeute an 1-Methyl-2-(p-chlorphenyl)-aziridin betrug 39 % (Selektivität 93 %).

### Beispiel 15

Brij 35 ® (90 mg) und 0,2 mmol Iod (254 mg) wurden zu 5 ml einer 1 molaren wässrigen Methylamin-Lösung hinzugefügt. Die Umsetzung wurde durch Zugabe von 0,5 mmol Styrol (57 µl) gestartet. Nach 2 Stunden Reaktionszeit bei Raumtemperatur wurde das Reaktionsgemisch mit Diethylether extrahiert. Die Ausbeute an 1-Methyl-2-phenylaziridin betrug 68 % (Selektivität 83 %).

### Beispiel 16

Brij 35 ® (90 mg) und 0,1 mmol Iod (127 mg) wurden zu 5 ml einer 1 molaren wässrigen Methylamin-Lösung hinzugefügt. Die Umsetzung wurde durch Zugabe von 0,48 mmol 2-Methyl-1-hepten (57 µl) gestartet. Nach 2 Stunden Reaktionszeit bei Raumtemperatur wurde das Reaktionsgemisch mit Diethylether extrahiert. Die Ausbeute an 1-Methyl-2-phenylaziridin betrug 3 % (Selektivität 83 %).

### Beispiel 17

Brij 35 ® (90 mg), Ammoniumiodid (73 mg) und 0,5 mmol Styrol (57 µl) wurden zu 5 ml einer 1 molaren wässrigen Methylamin-Lösung hinzugefügt. Die Umsetzung wurde durch die portionsweise Zugabe einer 10 bis 13 gew.-%igen wässrigen Natriumhypochlorit-Lösung (400 µl) gestartet, wobei jeweils 40 µl pro 5 Minuten zugegeben wurden. Nach 2 Stunden Reaktionszeit bei Raumtemperatur wurde das Reaktionsgemisch mit Diethylether extrahiert. Die Ausbeute an 1-Methyl-2-phenylaziridin betrug 39 % (Selektivität 95 %).

### Beispiel 18

Brij 35 ® (90 mg), Ammoniumiodid (73 mg) und 0,5 mmol Styrol (57 µl) wurden zu 5 ml einer 1 molaren wässrigen Methylamin-Lösung hinzugefügt. Die Umsetzung wurde durch die portionsweise Zugabe einer 10 bis 13 gew.-%igen wässrigen Natriumhypochlorit-Lösung (1 ml) gestartet, wobei jeweils 100 µl pro 5 Minuten zugegeben wurden. Nach 2 Stunden Reaktionszeit bei Raumtemperatur wurde das Reaktionsgemisch mit Diethylether extrahiert. Die Ausbeute an 1-Methyl-2-phenylaziridin betrug 73 % (Selektivität 97 %).

### Beispiel 19

Brij 35 ® (90 mg), Ammoniumiodid (73 mg) und 0,5 mmol Styrol (57 µl) wurden zu 5 ml einer 0,5 molaren wässrigen Methylamin-Lösung hinzugefügt. Die Umsetzung wurde durch die portionsweise Zugabe einer 10 bis 13 gew.-%igen wässrigen Natriumhypochlorit-Lösung (1 ml) gestartet, wobei jeweils 100 µl pro 5 Minuten zugegeben wurden. Nach 2 Stunden Reaktionszeit bei Raumtemperatur wurde das Reaktionsgemisch mit Diethylether extrahiert. Die Ausbeute an 1-Methyl-2-phenylaziridin betrug 52 % (Selektivität 95 %).

### Beispiel 20

Brij 35 ® (90 mg), Ammoniumiodid (73 mg) und 0,5 mmol Styrol (57 µl) wurden zu 5 ml einer 0,6 molaren wässrigen Ethylamin-Lösung hinzugefügt. Die Umsetzung wurde durch die portionsweise Zugabe einer 10 bis 13 gew.-%igen wässrigen Natriumhypochlorit-Lösung (1 ml) gestartet, wobei jeweils 100 µl pro 5 Minuten zugegeben wurden. Nach 2 Stunden Reaktionszeit bei Raumtemperatur wurde das Reaktionsgemisch mit Diethylether extrahiert. Die Ausbeute an 1-Ethyl-2-phenylaziridin betrug 55 % (Selektivität 96 %).

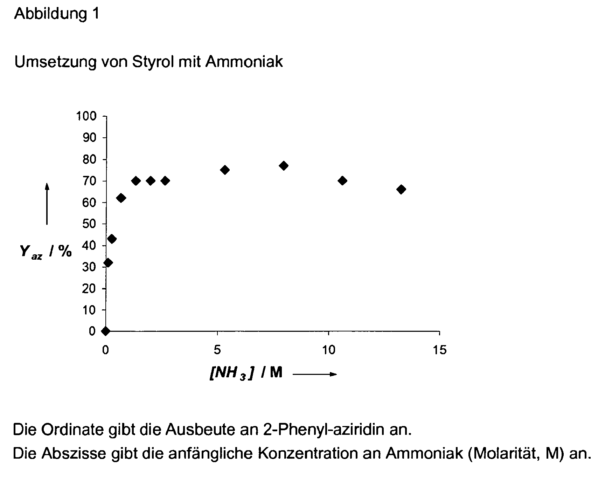

Die folgende Abbildung 1 zeigt beispielhaft bezüglich der erfindungsgemäßen Umsetzung von Styrol mit Ammoniak (NH₃) die Abhängigkeit der Ausbeute an 2-Phenylaziridin von der anfänglichen Ammoniak-Konzentration. Die Bedingungen der Versuche entsprachen denen des Beispiels 6, mit dem Unterschied, dass die NH₃-Konzentration variiert wurde. Wie ersichtlich, liegt der gefundene bevorzugte Bereich der Ammoniak-Konzentration bei ≥ 1,2 bis 15 molar.

Im Gegensatz dazu liegt überraschend der gefundene bevorzugte Bereich der primären Amin - Konzentration bei erfindungsgemäßen Umsetzungen mit primären Aminen (R⁵NH₂) bei > 0,5 bis ≤ 1,1 molar. Vergleiche hierzu auch die Beispiele 6 und 19.

## Patentansprüche

1. Verfahren zur Herstellung eines N-substituierten Aziridins der Formel III 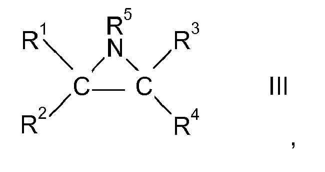 **dadurch gekennzeichnet, dass** man ein Olefin der Formel I 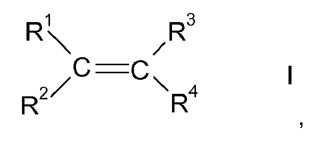 wobei R¹ bis R⁴ unabhängig voneinander Wasserstoff und R¹ bis R⁵ unabhängig voneinander lineare oder verzweigte Alkylreste mit 1 bis 16 Kohlenstoffatomen, Hydroxyalkylreste mit 1 bis 4 Kohlenstoffatomen, Cycloalkylreste mit 5 bis 7 Kohlenstoffatomen, Benzylreste und Phenylreste, die jeweils in o-, m- oder p-Stellung des Phenylrestes durch Methoxy-, Hydroxy-, Chlor- oder Alkylreste mit 1 bis 4 Kohlenstoffatomen substituiert sein können, darstellen und die Reste R¹ oder R² mit den Resten R³ oder R⁴ zu einem 5- bis 12-gliedrigen Ring geschlossen sein können oder die Reste R¹ und R² zu einem 5- bis 12-gliedrigen Ring geschlossen sein können, mit einem primären Amin der Formel R⁵NH₂ in Gegenwart von Iod oder Brom umsetzt, wobei die Konzentration des primären Amins (R⁵NH₂) im Reaktionsgemisch kleiner oder gleich 1,1 molar (≤ 1,1 M) beträgt, und man die Aziridin-Herstellung in Gegenwart einer oberflächenaktiven Substanz durchführt.

2. Verfahren zur Herstellung eines Aziridins der Formel III (Formel wie in Anspruch 1 definiert), **dadurch gekennzeichnet, dass** man ein Olefin der Formel I (Formel wie in Anspruch 1 definiert) mit einem primären Amin der Formel R⁵NH₂ in Gegenwart eines Iodids und eines Oxidationsmittels, das das Iodid zu Iod zu oxidieren vermag, umsetzt, wobei die Konzentration des primären Amins (R⁵NH₂) im Reaktionsgemisch kleiner oder gleich 1,1 molar (≤ 1,1 M) beträgt und wobei man als Oxidationsmittel Sauerstoff, Wasserstoffperoxid, Alkylhydroperoxid, Anthrachinonendoperoxid, unterchlorige Säure, tert.-Butylhypochlorit, tert.-Butylhypobromit, tert.-Butylhypoiodit, Distickstoffmonoxid, ein Alkalimetall- oder ein Erdalkalimetallhypochlorit verwendet oder man als Oxidationsmittel eine Anode einsetzt (elektrochemische Oxidation).

3. Verfahren zur Herstellung eines Aziridins der Formel III (Formel wie in Anspruch 1 definiert), **dadurch gekennzeichnet, dass** man ein Olefin der Formel I (Formel wie in Anspruch 1 definiert) mit einem primären Amin der Formel R⁵NH₂ in Gegenwart eines Bromids und eines Oxidationsmittels, das das Bromid zu Brom zu oxidieren vermag, umsetzt, wobei die Konzentration des primären Amins (R⁵NH₂) im Reaktionsgemisch kleiner oder gleich 1,1 molar (≤ 1,1 M) beträgt und wobei man als Oxidationsmittel Sauerstoff, Wasserstoffperoxid, Alkylhydroperoxid, Anthrachinonendoperoxid, unterchlorige Säure, tert.-Butylhypochlorit, tert.-Butylhypobromit, tert.-Butylhypoiodit, Distickstoffmonoxid, ein Alkalimetall- oder ein Erdalkalimetallhypochlorit verwendet oder man als Oxidationsmittel eine Anode einsetzt (elektrochemische Oxidation).

4. Verfahren nach einem der drei vorhergehenden Ansprüche, **dadurch gekennzeichnet, dass** die Konzentration des primären Amins (R⁵NH₂) im Reaktionsgemisch bei Reaktionsbeginn größer als 0,5 molar (> 0,5 M) beträgt.

5. Verfahren nach Anspruch 2 oder 4 (rückbezogen auf Anspruch 2), **dadurch gekennzeichnet, dass** man als Iodid ein Alkali-, Erdalkali-, Ammonium-, Tetraalkylammoniumiodid oder Gemische dieser Iodide (zwei oder mehrere) verwendet.

6. Verfahren nach Anspruch 3 oder 4 (rückbezogen auf Anspruch 3), **dadurch gekennzeichnet, dass** man als Bromid ein Alkali-, Erdalkali-, Ammonium-, Tetraalkylammoniumbromid oder Gemische dieser Bromide (zwei oder mehrere) verwendet.

7. Verfahren nach den Ansprüchen 2, 4 (rückbezogen auf Anspruch 2) oder 5, **dadurch gekennzeichnet, dass** man das Iodid als Gemisch aus Iodid und Iod einsetzt.

8. Verfahren nach den Ansprüchen 3, 4 (rückbezogen auf Anspruch 3) oder 6, **dadurch gekennzeichnet, dass** man das Bromid als Gemisch aus Bromid und Brom einsetzt.

9. Verfahren nach einem der Ansprüche 2, 3, 4 (rückbezogen auf Ansprüche 2 oder 3), 5 bis 8, **dadurch gekennzeichnet, dass** man die Aziridin-Herstellung in Gegenwart einer oberflächenaktiven Substanz durchführt.

10. Verfahren nach einem der vorhergehenden Ansprüche, **dadurch gekennzeichnet, dass** man die Aziridin-Herstellung in Gegenwart einer nichtionischen oberflächenaktiven Substanz durchführt.

11. Verfahren nach dem vorhergehenden Anspruch, **dadurch gekennzeichnet, dass** man einen Polyalkylenglykolalkylether oder einen ethoxylierten Fettalkohol, ein ethoxyliertes Alkylphenol oder ein ethoxyliertes Fettamin als nichtionische oberflächenaktive Substanz verwendet.

12. Verfahren nach einem der vorhergehenden Ansprüche, **dadurch gekennzeichnet, dass** man die Umsetzung in Gegenwart von Wasser durchführt.

13. Verfahren nach einem der Ansprüche 1 bis 11, **dadurch gekennzeichnet, dass** man die Umsetzung in Gegenwart von Wasser und einem organischen Lösungsmittel durchführt.

14. Verfahren nach dem vorhergehenden Anspruch, **dadurch gekennzeichnet, dass** man als organisches Lösungsmittel einen Ether oder einen aliphatischen, cycloaliphatischen oder aromatischen Kohlenwasserstoff verwendet.

15. Verfahren nach einem der vorhergehenden Ansprüche, **dadurch gekennzeichnet, dass** man die Aziridin-Herstellung in Gegenwart eines Zeoliths durchführt.

16. Verfahren nach einem der vorhergehenden Ansprüche, **dadurch gekennzeichnet, dass** das Molverhältnis von Olefin (I) zu primärem Amin (R⁵NH₂) zu Halogenid, Halogen oder Halogenid + Halogen 1 : 1 bis 100 : 0,001 bis 1,5 beträgt.

17. Verfahren nach einem der vorhergehenden Ansprüche, **dadurch gekennzeichnet, dass** man die Herstellung des Aziridins bei einer Temperatur im Bereich von 0 °C bis 300 °C durchführt.

18. Verfahren nach einem der vorhergehenden Ansprüche, **dadurch gekennzeichnet, dass** man die Herstellung des Aziridins bei einem Absolutdruck im Bereich von 1 bis 300 bar durchführt.

19. Verfahren nach einem der Ansprüche 1, 4 (rückbezogen auf Anspruch 1), 10 bis 18 (jeweils nicht rückbezogen auf Ansprüche 2 oder 3), **dadurch gekennzeichnet, dass** man die Herstellung des Aziridins in zwei Teilschritten durchführt, wobei im ersten Teilschritt die Umsetzung des Olefins (I) mit Ammoniak bzw. primärem Amin (R⁵NH₂) in Gegenwart von Iod oder Brom erfolgt und im zweiten Teilschritt das im ersten Teilschritt gebildete Halogenid wieder zum entsprechenden Halogen oxidiert und in den ersten Teilschritt zurückgeführt wird.

20. Verfahren nach einem der vorhergehenden Ansprüche zur Herstellung von N-Methyl-aziridin bzw. N-Ethyl-aziridin, **dadurch gekennzeichnet, dass** man Ethylen mit Monomethylamin bzw. Monoethylamin umsetzt.

21. Verfahren nach einem der Ansprüche 1 bis 19 zur Herstellung von 1,2-Dimethylaziridin bzw. 1-Ethyl-2-methyl-aziridin, **dadurch gekennzeichnet, dass** man Propylen mit Monomethylamin bzw. Monoethylamin umsetzt.

22. Verfahren nach einem der Ansprüche 1 bis 19 zur Herstellung von 1,2,2-Trimethyl-aziridin bzw. 1-Ethyl-2,2-dimethyl-aziridin, **dadurch gekennzeichnet, dass** man Isobuten mit Monomethylamin bzw. Monoethylamin umsetzt.
